# EUROPEAN PATENT SPECIFICATION

(11) **EP 2 217 164 B1**
(45) Date of publication and mention of the grant of the patent: **13.03.2019**
(21) Application number: 08848946.3
(22) Date of filing: 03.11.2008
(51) Int. Cl.: A61B 17/82, F16G 11/04

(54) **MINIMALLY INVASIVE CERCLAGE SYSTEM**
MINIMALINVASIVES CERCLAGE SYSTEM
SYSTÈME DE CERCLAGE PEU INVASIF

(30) Priority: 13.11.2007 US 996338 P
(43) Date of publication of application: 18.08.2010
(73) Proprietor: Synthes GmbH, 4436 Oberdorf (CH)
(72) Inventor: DELL'OCA, Alberto A. Fernandez, Montevideo 11500 (UY)
(74) Representative: Klunker IP Patentanwälte PartG mbB
(86) International application number: PCT/US2008/082231
(87) International publication number: WO 2009/064628

(56) References cited:
- WO-A-2004/019797
- WO-A-2005/124187
- US-A- 5 908 421
- US-A1- 2006 212 073

## Description

### Field of the Invention

The invention relates generally to an apparatus for the treatment of bones and more particularly relates to an apparatus for a minimally invasive bone cerclage technique.

### Background Information

Bone cerclage techniques generally involve the wrapping of a wire or other cable around a target portion of bone and the fixing of the cable in position to aid bone fixation and/or repair. Present bone cerclage devices and methods require at least one incision adjacent to the target portion of bone large enough to permit the insertion of tools used to insert and secure the cable around the bone.

US 5,908,421 A discloses a surgical device for fixing bone elements, having a crimp body and a cerclage cable that can be secured in the crimp body. The crimp body has two parallel lumens, in one of which the cable is fixedly secured. The cable is slidably received in the other lumen and can be temporarily secured by means of a tapered insert that creates a wedge effect in one direction, while the cable is still movable in the opposite direction. Final fixation is achieved by crimping the insert over the cable by means of a known forceps.

Other devices and methods for fixing bone elements by means of a surgical cable and a fixation element, like a clamping body, are disclosed in WO 2004/019797 A2, US 2006/0212073 A1 and WO 2005/124187 A2.

### Summary of the Invention

The present invention is directed to an apparatus for securing a cerclage member about a bone within a living body having the features defined in claim 1. The apparatus particularly comprises a crimp body including a first lumen extending longitudinally therethrough from a first proximal opening to a first distal opening, the first lumen being sized and shaped to slidably receive a cerclage member therein and a second lumen extending substantially parallel to the first lumen from a second proximal opening to a second distal opening, a diameter of second distal opening and a distal portion of the second lumen being greater than a diameter of a proximal portion of the second lumen and an insert sized to be inserted into the distal portion of the second lumen, an insert lumen extending through the insert aligning with the second proximal opening of the second lumen when the insert is received within the distal portion of the second lumen, a distal head of the insert including an outer surface tapered from a maximum diameter at a proximal end thereof to a minimum diameter at a distal end thereof, the insert being crushable over a cerclage member inserted therethrough to lock the cerclage member relative to the crimp body and maintaining a desired tension on the cerclage member.

The present disclosure further includes an apparatus for securing a cerclage member about a bone within a living body comprising a first crimp member including first and second lumens extending longitudinally therethrough, the first and second lumens being sized and shaped to slidably receive a cerclage member therein and a second crimp member defining third and fourth lumens extending therethrough, the third and fourth lumens being positioned within the second crimp member so that, when the second crimp member is coupled to the first crimp member in an initial configuration, the first lumen aligns with the third lumen and the second lumen aligns with the fourth lumen in combination with a driving member selectively moving the second crimp member relative to the first crimp member from the initial configuration to a locking configuration in which the first lumen is moved out of alignment with the third lumen to lock the cerclage member relative to the first and second crimp members, maintaining a desired tension on the cerclage member.

The present disclosure further includes an apparatus for securing a cerclage member about a bone within a living body comprising a crimp body including a first lumen extending longitudinally therethrough from a first proximal opening to a first distal opening, the first lumen being sized and shaped to slidably receive a cerclage member therein and a second lumen extending longitudinally through the crimp body between a second proximal opening to a second distal opening, the crimp body further including a driving member lumen extending from an outer opening open to an exterior of the crimp body and an inner opening opening into the second lumen and an insert slidable into the second lumen, the insert defining a third lumen having a diameter sufficient to slidably receive a cerclage member therein in combination with a driving member movable within the driving member lumen and lockable at a desired position therein, the driving member being movable between an initial position and a locking position in which the driving member extends into the second lumen crushing the insert to lock a cerclage member received in the third lumen in place within the crimp body at a desired tension.

### Brief Description of the Drawings

Fig. 1 shows an exploded view of a crimp according to an embodiment of the present invention;
Fig. 2 shows a first partial cross-sectional view of the crimp of Fig. 1 in an unlocked configuration;
Fig. 3 shows a second partial cross-sectional view of the crimp of Fig. 1 in a locked configuration;
Fig. 4 shows a perspective view of a crimp according to an example which is not part of the present invention;
Fig. 5 shows a first partial cross-sectional view of the crimp of Fig. 4 in an unlocked configuration;
Fig. 6 shows a second partial cross-sectional view of the crimp of Fig. 4 in a locked configuration;
Fig. 7 shows a perspective view of a crimp according to an example which is not part of the present invention;
Fig. 8 shows a first partial cross-sectional view of the crimp of Fig. 7 in an unlocked configuration;
Fig. 9 shows a second partial cross-sectional view of the crimp of Fig. 7 in a locked configuration;
Fig. 10 shows a perspective view of a crimp according to an example which is not part of the present invention;
Fig. 11 shows a perspective view of a first component of the crimp of Fig. 10;
Fig. 12 shows a perspective view of a second component of the crimp of Fig. 10;
Fig. 13 shows a perspective view of a third component of the crimp of Fig. 10;
Fig. 14 shows a first partial cross-sectional view of the crimp of Fig. 11 in an unlocked configuration;
Fig. 15 shows a second partial cross-sectional view of the crimp of Fig. 11 in a locked configuration;
Fig. 16 shows a perspective view of a crimp according to an example which is not part of the present invention;
Fig. 17 shows a first partial cross-sectional view of the crimp of Fig. 16 in an unlocked configuration;
Fig. 18 shows a second partial cross-sectional view of the crimp of Fig. 16 in a locked configuration;

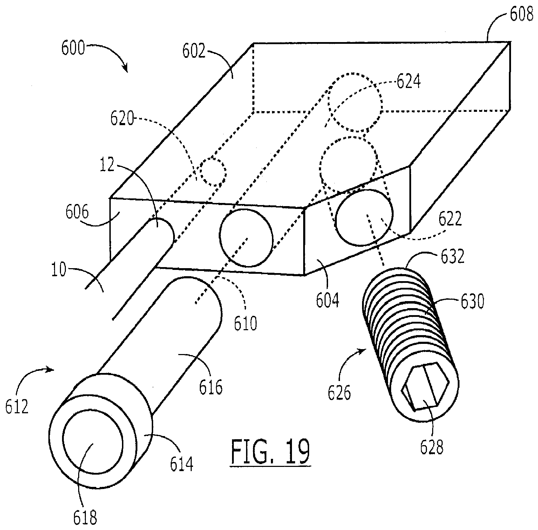

Fig. 19 shows a perspective view of a crimp according to an example which is not part of the present invention;
Fig. 20 shows a first partial cross-sectional view of the crimp of Fig. 19 in an unlocked configuration; and
Fig. 21 shows a second partial cross-sectional view of the crimp of Fig. 19 in a locked configuration.

### Detailed Description

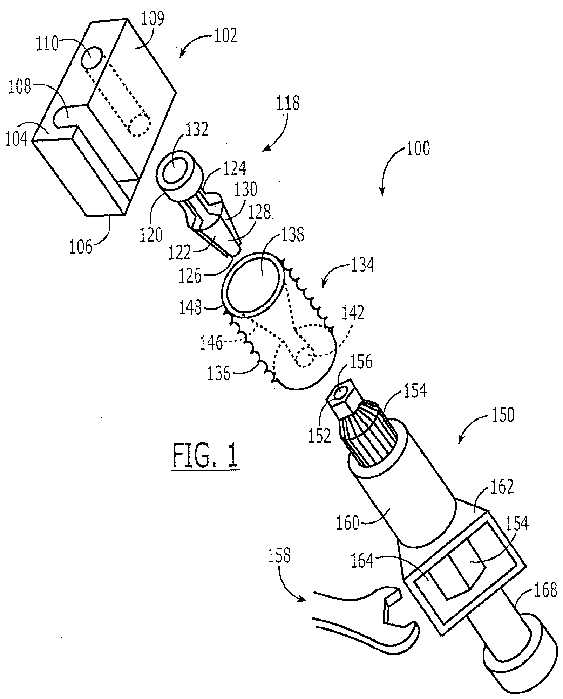

The present invention may be further understood with reference to the following description and the appended drawings, wherein like elements are referred to with the same reference numerals. The present invention relates generally to devices for the stabilization and fixation of fractured bones and bone fragments via a bone cerclage. Specifically, the present invention relates to devices for crimping a cerclage wire or cable looped around a target portion of a bone. The invention is shown in Figs. 1-3. Embodiments of the present invention may be employed with any of a plurality of procedures involving bone cerclage. Those skilled in the art will understand that, as used in this application, the term proximal refers to a direction along a length of the cerclage cable toward a first end thereof-e.g., an end including an enlarged projection which abuts a surface of the crimp to prevent the cable from being pulled distally therethrough. Distal then refers to the direction along the cable toward the other end thereof.

A crimping device 100 according to an exemplary embodiment of the present invention comprises a crimp body 102 for receiving proximal and distal ends of a cable 10 to be looped around a target portion of a bone (not shown). The crimp body 102 comprises a groove 108 extending longitudinally therethrough from a proximal face 104 to a distal face 106. The groove 108 is formed in a first face 109 of the crimp body 102 which, when the crimp body 102 is placed on a target portion of bone in the desired position, faces away from the bone. The groove 108 may, for example, be formed as a partially cylindrical depression with an opening in the first face being made smaller than a diameter of a cable 10 to be inserted therethrough so that the cable 10 can not slip out of the groove 108. Furthermore, since the groove 108 is open to the first face 109, the cable 10 comprising an enlarged diameter proximal end 12, can easily be inserted and removed from engagement therewith during an exemplary method of the present invention, as will be described in greater detail hereinafter.

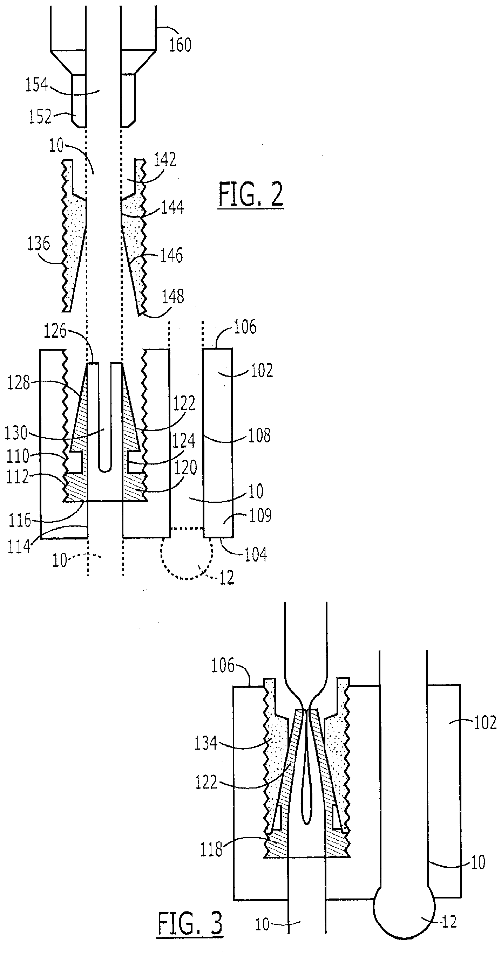

A lumen 110 extends longitudinally through the crimp body 102 substantially parallel to the groove 108. As shown in greater detail in Fig. 2, the lumen 110 comprises a first threaded region 112 and a second non-threaded region 114 proximal of the first threaded region 112 and separated therefrom another along a longitudinal axis of the lumen 110. The threaded region 112 extends proximally from the distal face 106 a predetermined distance to an end point 116 and has a diameter sufficient to receive an insert 118 therein, as will be described in greater detail hereinafter. The non-threaded region 114 extends distally from the proximal face 104 to the end point 116. The location of the end point 116 is selected to permit full insertion of the insert 118 into the threaded region 112, as will be described in greater detail hereinafter. The non-threaded region 114 is formed with a substantially circular cross-section and has a diameter sufficient to slidably receive the cable 10 therethrough while preventing the insertion of the insert 118 thereinto. It is noted, however, that the cross-section of the lumen 110 may be of any shape suited to a shape of the cable 10. In an exemplary embodiment, a diameter of the non-threaded region 114 is slightly larger than an outer diameter of the cable 10.

The insert 118 according to the first embodiment of the present invention is formed as an elongated hollow nail comprising an increased diameter head 120 at a proximal end thereof and a shaft 122 extending distally therefrom. An outer surface of the head 120 includes a thread sized and shaped to engage the threaded region 112 of the lumen 110. The shaft 122 is longitudinally separated from the head 120 by a cylindrical extension 124. A diameter of the shaft 122 tapers from a maximum diameter at a proximal end thereof to a minimum diameter at a distal tip 126 of the insert 118. A lumen 132 extending longitudinally through the insert 118 and open at both ends thereof, is sized and shaped to receive the cable 10 slidably therethrough. Specifically, a diameter of the lumen 132 may be slightly larger than an outer diameter of the cable 10. The shaft 122 further comprises a plurality of arms 128 spaced from one another by a plurality of slots 130 extending longitudinally along a length of the shaft 122. The insert 118 is formed of a material sufficiently flexible to permit the arms 128 to bend radially inward upon application thereto of a radially compressive force (as will be described in greater detail below) without fracturing. Exemplary material for the insert 118 may comprise any biocompatible or implantable material including, but not limited to, stainless steel, titanium, titanium alloys, Polyetheretherketone ("PEEK") or any resorbable material known in the art, as those skilled in the art will understand.

The tapered distal end of the insert 118 is sized and shaped to be selectively insertable into a correspondingly tapered lumen 138 extending longitudinally through a screw 134. An outer surface 136 of the screw 134 is threaded to engage threads of the threaded region 112 of the lumen 110. The lumen 138 tapers down in size in a distal direction from a maximum diameter at a proximal end 148 thereof to a minimum diameter at a taper end point 144. A cross-section of the lumen 138 at the taper end point 144 may be substantially circular with a diameter substantially equal to that of the lumen 132 so that the cable 10 may be slid therethrough as well. A socket 142 formed at a distal end of the screw 134 has a diameter greater than that of the lumen 138 at the taper end point 144 and is sized and shaped to receive a distal end of a screwdriver tip 152 to permit screwing of the screw 134 into the threaded region 112 of the lumen 110. Accordingly, the socket 142 may comprise any shape known in the art that will accommodate a screwdriver tip or other similar tool, as would be understood by those skilled in the art. In the embodiment shown, the socket 142 comprises a hexagonal shape to receive a hexagonal tip 152 of a screwdriver 150. A body 154 of the screwdriver 150 may also have a substantially hexagonal cross-section to permit manipulation thereof by a hex-head of a wrench 158. Alternatively, the body 154 of the screwdriver 150 may comprise any shape suitable for manipulation by the wrench 158 or other suitable tool. The screwdriver 150 further comprises a lumen 156 extending therethrough from a distal end comprising the tip 152 to a proximal end (not shown) attached to a tension device 168 sized to slidably receive the cable 10 therethrough.

The body 154 of the screwdriver 150 may further comprise an increased diameter handle 160 to aid in gripping and manipulation of the screwdriver 150. As would be understood by those skilled in the art, the handle 160 may be formed with any cross-sectional shape and, in one embodiment, may comprise an ergonomic shape to aid in gripping. Furthermore, an outer diameter of the handle 160 is sized and shaped to permit insertion thereof into a crimp holder 162. The crimp holder 162 is a fitting sized and shaped to slide over a proximal end of the screwdriver 150 and comprises a window 164 formed into a quadrant thereof to permit access to the screwdriver 150. Accordingly, manipulation of the screwdriver 150 by the wrench 158 may be performed through the window 164, as will be described in greater detail hereinafter. A proximal end of the crimp holder 162 abuts the tension device 168. The tension device 168 includes a lumen (not shown) extending longitudinally therethrough sized and shape to frictionally engage the cable 10. Specifically, a diameter of the lumen is slightly smaller than an outer diameter of the cable 10 to apply a tension to the cable 10 as it is advanced therethrough.

Figs. 2 - 3 sequentially depict the process by which the cable 10 is locked onto the crimp body 102. Initially, the cable 10 is looped around a target portion of bone in a desired position and the crimp body 102 is then placed at a desired position on the bone. A proximal portion of the cable 10 is then laid into the groove 108 and drawn proximally therethrough until the enlarged diameter proximal end 12 of the cable 10 engages the proximal face 104 at the opening of the groove 108 therein preventing the cable 10 from being pulled further distally into the groove 108. The distal end of the cable 10 which has been looped around the target portion of bone is then inserted into the lumen 110 via an opening thereto formed in the proximal face 104 and passed therethrough and out of the distal face 106. In passing through the lumen 110, the cable 10 passes through the lumen 132 of the insert 118 which resides within the lumen 110. The distal end of the cable 10 is then passed through the screw 134 and through the screw driver 150 and the crimp holder 162 to the tension device 168. The tension device 168 is then operated as would be understood by those skilled in the art to draw the cable 10 tight around the target portion of bone and to increase the tension thereon until a desired level of tension is reached. At this point, the screw 134 is screwed into the lumen 110 through operation of the screw driver 150 in the socket 142. As the screw 134 advances proximally into the lumen 110 over the tapered surface of the insert 118, the narrowing of the lumen 138 applies increasing radially inwardly directed pressure against the outer surface of the insert 118 until the insert 118 is crushed collapsing the arms 128 into the lumen 132 against the cable 10. This locks the cable 10 within the crimp body 102 preventing movement of the cable 10 relative thereto and maintaining the desired tension on the cable 10, as shown in Fig. 3.

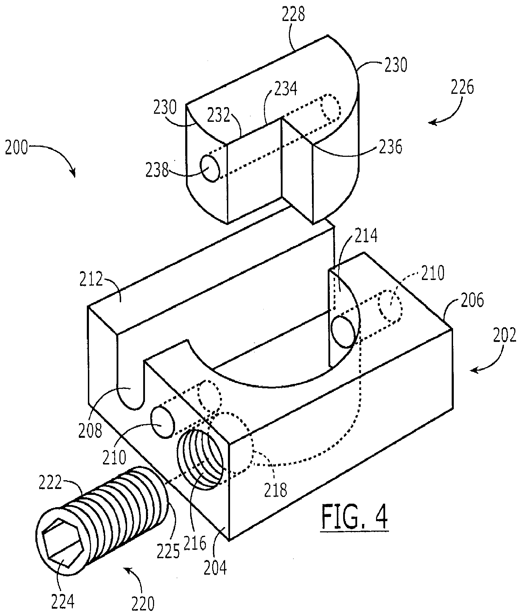

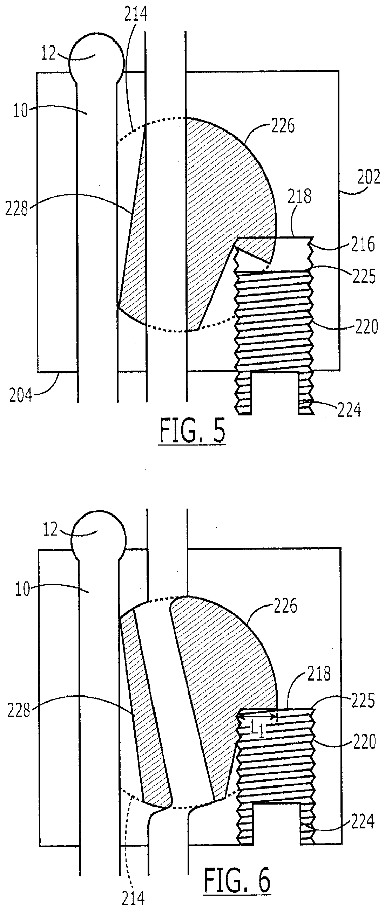

As shown in Figs. 4 - 6, a device 200 according to an illustrative example comprises a crimp body 202 including multiple bores extending therethrough to lock a cable 10 in position. The crimp body 202 is formed substantially similarly to the crimp body 102 with a groove 208 extending from a distal face 204 to a proximal face 206 and a cable lumen 210 extending longitudinally parallel thereto. The groove 208 and the cable lumen 210 are sized and shaped substantially similarly to the groove 108 and lumen 110 of Figs. 1 - 3 to slidably receive a cerclage cable 10 therethrough. The groove 208 is formed on a first lateral face 212 of the crimp body 202, which further comprises a recess 214 extending into the crimp body 202 from the lateral face 212. The recess 214 is substantially cylindrical and opens to both the groove 208 and the cable lumen 210 such that the cable 10, when advanced through the cable lumen 210, passes through the recess 214. A depth of the recess 214 is sufficient to permit the cable lumen 210 to open thereinto, as shown in detail in Fig. 4 and is, for example, at least as deep as the cable lumen 210 and the groove 208. The crimp body 202 further comprises a threaded hole 216 extending proximally a distance L₁ from the distal face 204 to open into at a distal end 218 thereof into the recess 214, as shown in Fig. 5. The threaded hole 216 is sized and shaped to receive a screw 220 therein. The screw 220 is formed as an insert with an elongated shaft 222 extending to a proximal end 225. A distal end of the screw 220 comprises a socket 224 sized and shaped to engage a screwdriver tip (not shown) or other tool to enable screwing thereof into the threaded hole 216. In the embodiment shown, the socket 224 is substantially hexagonal to enable screwing by a hex-head screwdriver, as those skilled in the art will understand. Alternatively, the socket 224 may comprise any shape known in the art to facilitate screwing by a screwdriver or other tool, as describe above with respect to Figs. 1 - 3.

An exemplary insert 226 according to this example is sized and shaped to apply locking pressure to the cable 10 when inserted into the groove 214 as will be described in more detail below. As shown in Fig. 4, the insert 226 comprises a lateral wall 228 and a wall 230 extending along a portion of a circle with a radius of curvature substantially matching a radius of curvature of the recess 214 so that the insert 226 is rotatable within the recess 214 and so that, when inserted therein, an outer surface of the insert 226 forms a substantially continuous surface with an outer surface of the crimp body 202. The lateral wall 228 is formed so that, when the insert 226 is oriented in an initial configuration, the wall 228 forms a surface of the groove 208 substantially continuous with portions thereof proximal and distal therefrom. A cut-out 232 is formed on the insert 226 on a side substantially opposite the planar wall 228 extending into the substantially circular portion of the insert 226 with a first wall 234 of the cut-out 232 extending substantially parallel to the groove 208 and a second wall 236 extending substantially perpendicular to the groove 208 for a length L₁. A thickness of the insert 226 is selected so that an insert lumen 238 may be formed therethrough oriented so that, when in the initial configuration, the insert lumen 238 aligns with the proximal and distal ends of the cable lumen 210. The second wall 236 is positioned, in the initial configuration, in alignment with an axis of the threaded hole 216 - i.e., in the initial configuration, the second wall 236 is substantially perpendicular to the threaded hole 216 and extends across at least a portion of the threaded hole 216.

Figs. 5 - 6 sequentially depict the process by which the cable 10 is locked onto the crimp body 202. As noted earlier, the cable 10 may first be looped around a target portion of bone to be stabilized and a device 200 which is, for example, factory assembled in the initial configuration shown in Fig. 5 is placed at a desired location adjacent to a bone to be treated. A proximal portion of the cable 10 is then laid into the groove 208 and drawn distally thereinto until the enlarged diameter proximal end 12 engages the proximal face 206 of the crimp body 202. The distal portion of the cable 10 which has been looped around the target portion of bone until the distal end thereof is again adjacent to the proximal face 206 of the crimp body 202 is then inserted into the cable lumen 210, passed distally through the insert lumen 238 and out of the distal end of the cable lumen 210. The cable 10 is then connected to any known tensioning mechanism and tightened to the desired tension as would be understood by those skilled in the art. As shown in Fig. 6, the screw 220 is then tightened into the threaded hole 216 moving the proximal end 225 of the screw 220 proximally against the second wall 236 of the insert 226 rotating the insert 226 as shown in Fig. 6 so that a distal end thereof is driven laterally past the distal portion of the cable lumen 210 toward the groove 208 while the proximal end of the insert lumen 238 is moved laterally past the proximal portion of the cable lumen 210 toward the screw 220 deforming the portions of the cable 10 received within the crimp body 202 and locking the cable 10 in position therein. This locking also maintains the desired tension on the cable 10 after the tensioning mechanism has been decoupled therefrom. The portion of the cable 10 extending from the crimp body 202 may then be clipped and this portion of the cable and any mechanisms, etc. used in the procedure may be removed from the body to complete the procedure.

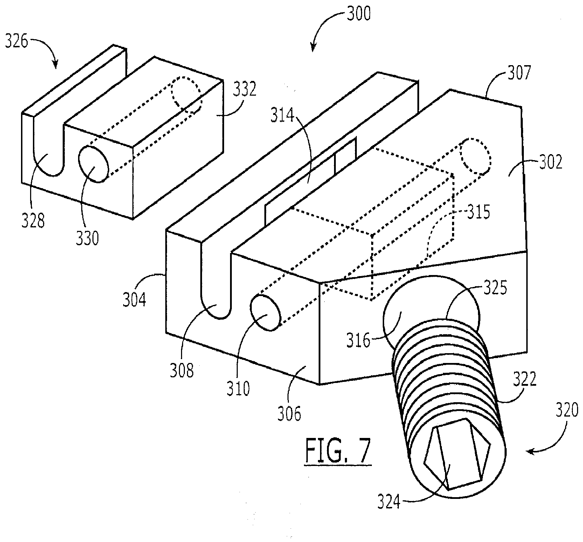

As shown in Figs. 7 - 9, a crimp body 302 of a device 300 according to an illustrative example includes an insert 326 defining an insert lumen 330 and an insert groove 328 extending longitudinally therethrough in alignment with a cable lumen 310 and a groove 308 of a crimp body 302. The crimp body 302 according to this embodiment is formed as a five-sided polygon and, in one embodiment, may be a pentagon. Alternatively, the crimp body 302 may be triangular, trapezoidal or may assume any suitable shape as would be understood by those skilled in the art. The groove 308 and the cable lumen 310 extend substantially parallel to one another through the crimp body 302 from a distal wall 306 to a proximal wall 307 thereof. The dimensions of the groove 308 and the cable lumen 310 are preferably substantially similar in relation to a cable 10 to be used therewith as described above in regard to the grooves and cable lumens of the above-described embodiments. A slot 314 which extends through the crimp body 202 laterally with respect to the groove 308 and the cable lumen 310 from a first lateral wall 304, with a thickness of the slot 314 being smaller than a thickness of the crimp body 302. The slot 314 has a size and shape corresponding to that of an insert 326 to be slid thereinto and, in this embodiment is illustrated as a substantially rectangular cavity adapted to receive the substantially rectangular insert 326 therein.

Specifically, the insert 326 includes an insert groove 328 and an insert lumen 330 extending longitudinally therethrough positioned so that, in an initial configuration, the groove 328 aligns with the groove 308 and the insert lumen 330 aligns with the cable lumen 310. As described above in regard to the crimp body 202, the insert groove 328 and the insert lumen 330 are sized shaped to slidably receive the cable 10 therethrough. When in the initial configuration, a first end 315 of the insert 326 extends into the crimp body 302 past the cable lumen 310 to project into a proximal end 325 of a threaded hole 316 extending into the crimp body 302 from a second lateral wall 309. A screw 320 is screwed into the threaded hole 316 so that, in the initial configuration, a distal end thereof projects distally from the second lateral wall 309 while a proximal end 325 thereof remains within a distal portion of the threaded hole 316 out of engagement with the first end 315 of the insert 326. The proximal end 325 of the screw 320 comprises a proximal face which, in the initial configuration, contacts the first end 315 of the insert 326 without moving the insert 326 out of alignment with the groove 308 and the cable lumen 310. For example, the proximal face of the screw 320 may taper at an angle corresponding to an angle between a longitudinal axis of the threaded hole 316 and an axis along which the insert 326 moves into and out of the insert lumen 330. A distal end of the screw 320 comprises a substantially hexagonal groove 324 (or other suitable screw tool receiving structure) sized and shaped to engage a screwdriver tip (not shown) to enable screwing thereof into and out of the threaded hole 316.

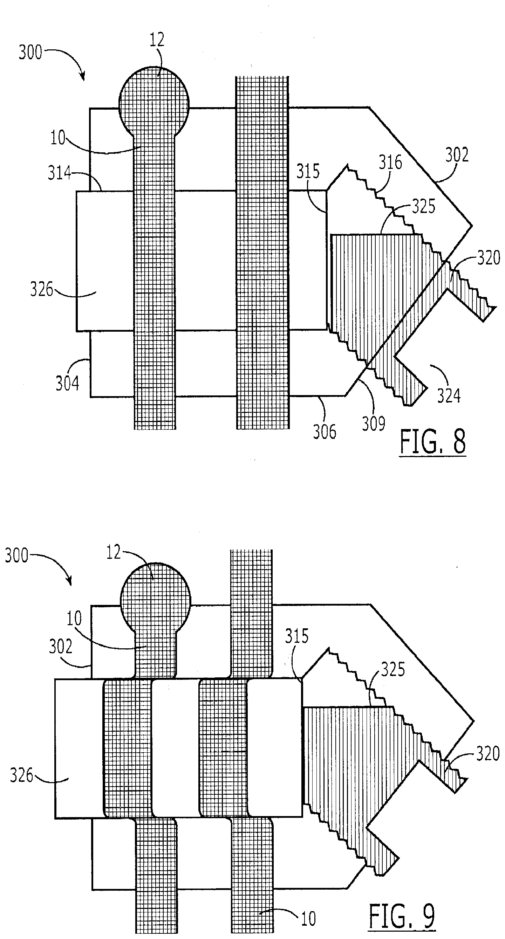

As noted earlier, the cable 10 may be looped around a target portion of bone to be stabilized and a device 300 which is, for example, factory assembled in the initial configuration shown in Fig. 8 is placed at a desired location adjacent to the bone. A proximal portion of the cable 10 is then laid into the groove 308 and drawn distally thereinto until the enlarged diameter proximal end 12 engages the proximal face 306 of the crimp body 302. The distal portion of the cable 10 which has been looped around the target portion of bone until the distal end thereof is adjacent to the proximal face 306 of the crimp body 302 is then inserted into the cable lumen 310, passed through the insert lumen 330 and out of the distal end of the cable lumen 310. The cable 10 is then connected to any known tensioning mechanism and tightened to the desired tension as would be understood by those skilled in the art. As shown in Fig. 9, the screw 320 is then tightened into the threaded hole 316 moving the proximal end 325 of the screw 320 proximally against the first end 315 of the insert 326 sliding the insert 326 laterally relative to the cable lumen 310 and the groove 308 as shown in Fig. 9 deforming the portions of the cable 10 received within the crimp body 302 and locking the cable 10 in position therein. This locking also maintains the desired tension on the cable 10 after the tensioning mechanism has been decoupled therefrom. The portion of the cable 10 extending from the crimp body 302 may then be clipped and this portion of the cable and any mechanisms, etc. used in the procedure may be removed from the body to complete the procedure.

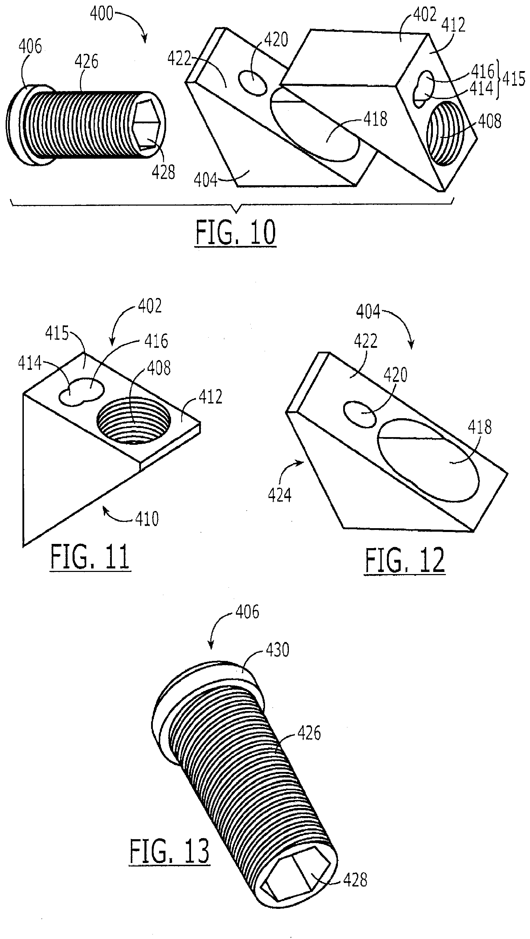

In yet another alternate example, as shown in Figs. 10 - 15, the crimp body comprises two separate elements that lockingly engage one another. Specifically, a device 400 comprises a first crimp member 402 and a second crimp member 404 which together comprise a crimp body when joined together via a screw 406. Specifically, each of the first and second crimp members 402, 404, respectively, are both substantially triangular with the first crimp member 402 including a threaded lumen 408 extending from an oblique surface 410 thereof to an opposite lateral wall 412. The threaded lumen 408 is sized and shaped to receive the screw 406 therethrough, with a diameter of the threaded lumen 408 being substantially equivalent to an outer diameter of a shaft 426 of the screw 406. Furthermore, the threaded lumen 408 extends substantially perpendicular to the lateral wall 412. A first cable lumen 416 extends through the first crimp member 402 substantially parallel to the threaded lumen 408. The first cable lumen 416 is sized and shaped to receive the cable 10 therethrough and is large enough to pass the enlarged proximal end 412 of the cable 10 therethrough. A second cable lumen 414 having a diameter smaller than the enlarged proximal end of the cable 10 but is formed adjacent to and opens into the first cable lumen 416. Thus, the first cable lumen 416 and the second cable lumen 414 together form a keyhole lumen 415 with a substantially figure-8 shaped cross-section comprising the overlapping non-concentric circles, as shown in Figs. 10 and 11.

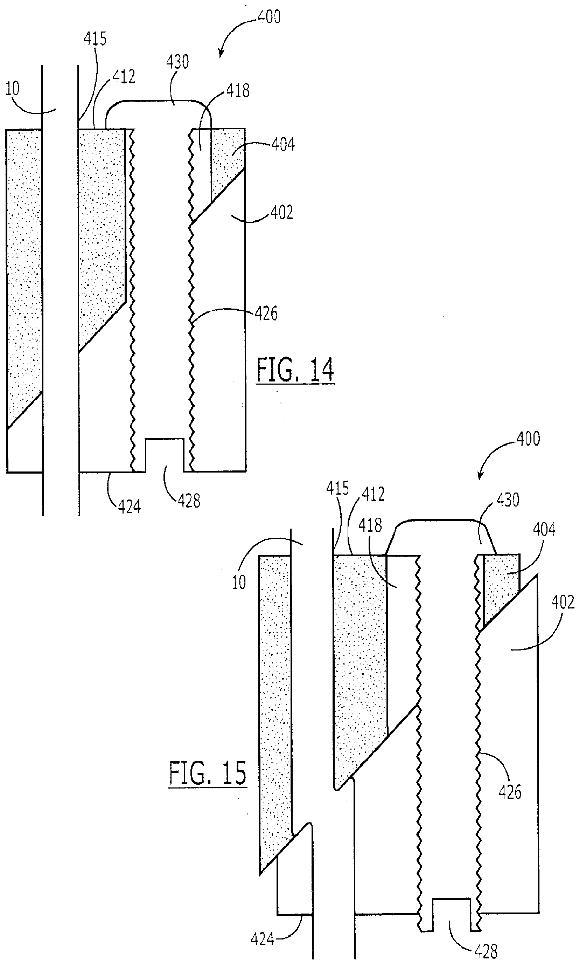

The second crimp member 404 comprises a third cable lumen 420 extending therethrough from an oblique surface 422 to an opposite lateral surface 424. The third cable lumen 420 is sized and shaped to receive two lengths of the cable 10 therethrough side by side as will be described in more detail below. A screw lumen 418 extends through the second crimp member 404 substantially parallel to the third cable lumen 420. The screw lumen 418 is substantially oval in cross-section and extends through the second crimp member 404 substantially perpendicularly to the opposite lateral surface 424. The screw lumen 418 is not threaded and has a width (an extent along the oblique surface 422) greater than a width of the threaded lumen 408 (i.e., an extent of the threaded lumen 408 across the oblique surface 410). The screw lumen 418 and the threaded lumen 408 are positioned within the second and first crimp members 404, 402, respectively, so that, when the oblique surfaces 410 and 422 are in contact with one another in an initial configuration, the screw lumen 418 extends over the entire threaded lumen and across a portion of the oblique surface 410 laterally outside the threaded lumen 408 with a first end of the screw lumen 418 in alignment with a first end of the threaded lumen 408 as shown in Fig. 14. In this initial configuration, the third cable lumen 420 aligns with the keyhole lumen 415 of the first crimp body 402. The screw 406 has a threaded shaft 426 extending from an increased diameter head 430 at a first end thereof to a socket 428 at a second end thereof. As discussed earlier, the socket 428 may comprise any shape designed to facilitate coupling the screw 406 to a tool for screwing the screw 406 into the crimp body 402 as will be described below. For example, the socket 428 may be formed in a substantially hexagonal shape to permit screwing of the screw 406 by a hexagonally shaped head of a screwdriver (not shown), as those skilled in the art will understand.

Figs. 14 - 15 sequentially depict the process by which the cable 10 is locked onto the first and second crimp members 402, 404. Initially, the cable 10 is looped around a target portion of bone to be stabilized and the first and second crimp members 402, 404 are positioned adjacent to the bone in a target location with the oblique surfaces 422 and 410 against one another with the lumens 415 and 420 aligned with one another and the screw lumen 418 over lying the threaded lumen 408 as shown in Fig. 14. The enlarged proximal end 12 of the cable 10 is then inserted into the lumen 420 and passed proximally therethrough into the first cable lumen 416 until the enlarged proximal end 12 exits the first cable lumen 416. A distal end of a cable 10 which has been looped around the bone is then inserted into the first cable lumen 416 and inserted therethrough next to the proximal portion of the cable 10. This pushes the proximal portion of the cable 10 laterally into the second cable lumen 414 so that the enlarged proximal end 12 of the cable 10 engages the portion of the crimp body 402 surrounding the second cable lumen 414 preventing the proximal portion of the cable 10 from being drawn proximally thereinto. The distal portion of the cable 10 is passed distally through the first cable lumen 416 into the third cable lumen 420 and coupled to a tensioning mechanism as described above. When the desired tension has been applied to the cable 10, the screw 406 is screwed into the threaded lumen 408 pulling the first crimp body 402 toward the head 430 of the screw 406 which forces the oblique surfaces 422 and 410 to slide over one another moving the third cable lumen 420 of the crimp body 404 laterally relative to the keyhole lumen 415 of the crimp body 402. This lateral movement of the third cable lumen 420 relative to the keyhole lumen 415 deforms the proximal and distal portions of the cable 10 extending between these lumens, locking the cable 10 against movement relative to the crimp bodies 402, 404 and maintaining the desired tension on the cable 10.

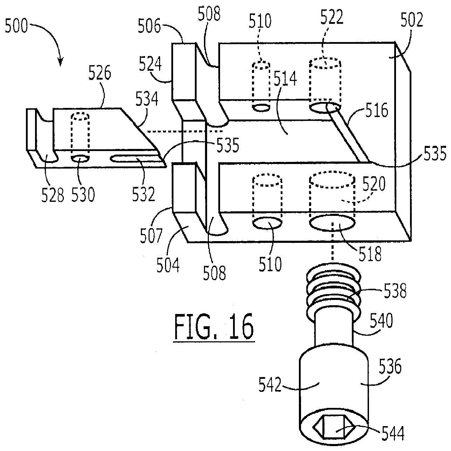

In yet another alternate example, a device 500 as shown in Figs. 16 - 18 is substantially similar to the device 300 of Figs. 7 - 9 and operates on similar principles. Specifically, in addition to an insert groove 528 and an insert lumen 530, an insert 526 of the device 500 also comprises a screw groove 532 extending longitudinally therethrough. A crimp body 502 is substantially rectangular in cross-section with a groove 508 and a cable lumen 510 extending longitudinally therethrough from a first lateral face 504 to a second lateral face 506. The groove 508 and the cable lumen 510 are dimensioned substantially to slidably receive a cerclage cable 10 as described above in regard to the groove 108 and the cable lumen 110 of the device of Figs. 1 - 3. The crimp body 502 further comprises a screw lumen 520 extending longitudinally therethrough substantially parallel to the groove 508 and the cable lumen 510 from a distal end comprising a threaded region 522 to a proximal end comprising a non-threaded region 518. A slot 514 extending into the crimp body 502 laterally across the groove 508 and the cable lumen 510 from a side wall 507 has a substantially rectangular cross-section with an wall 516 at a distal end thereof extending at an oblique angle with respect to the axes of the groove 508 and the cable lumen 510. The slot 514 does not extend entirely through the crimp body 502 having a depth, for example, slightly greater than a depth of the groove 508 (e.g., deeper by an amount sufficient to permit the insert lumen 530 to be formed therethrough in alignment with the cable lumen 510). The insert includes an oblique end 534 angled so that, as the insert 526 is inserted into the slot 514, the oblique end 534 is substantially parallel to the wall 516. The insert 526 is sized so that, when in an initial configuration, with the oblique end 534 adjacent to the wall 516 the insert groove 528 and the insert lumen 530 are aligned with the groove 508 and the cable lumen 510, respectively. In addition, in the initial configuration, an end of the screw groove 532 closest to the insert lumen 530 is aligned with the screw lumen 520. In one exemplary embodiment, the oblique end 534 and the wall 516 are formed at an angle between 0 - 89° with respect to the groove 508 and the cable lumen 510. A width of the insert 526 (i.e., a distance across the insert 526 in a direction parallel to the cable lumen 510) is smaller than a width of the slot 514 so that the insert 526 is movable along an axis parallel to the cable lumen 510 within the slot 514. A length of the insert 526 may be substantially equivalent to a length of the slot 514 so that when a distal tip 535 lies adjacent to the distal tip 515 of the slot 514, the insert 526 lies completely within the confines of the slot 514.

The screw 536 is formed as an insert with an elongated shaft 540 comprising an increased diameter head 542 at a proximal end and an increased diameter threaded portion 538 at a distal end. The head 542 also comprises a slot 544 sized and shaped to engage a tip of a screwdriver, as described in greater detail in regard to the earlier embodiments. Furthermore, as would be understood by those skilled in the art, each of the head 542, the shaft 540 and the threaded portion 538 may have a substantially circular cross-section as described above to facilitate rotation in the screw lumen 520, the groove 532 and the . Furthermore, a diameter of the head 542 is greater than a diameter of the screw groove 532 formed in the insert.

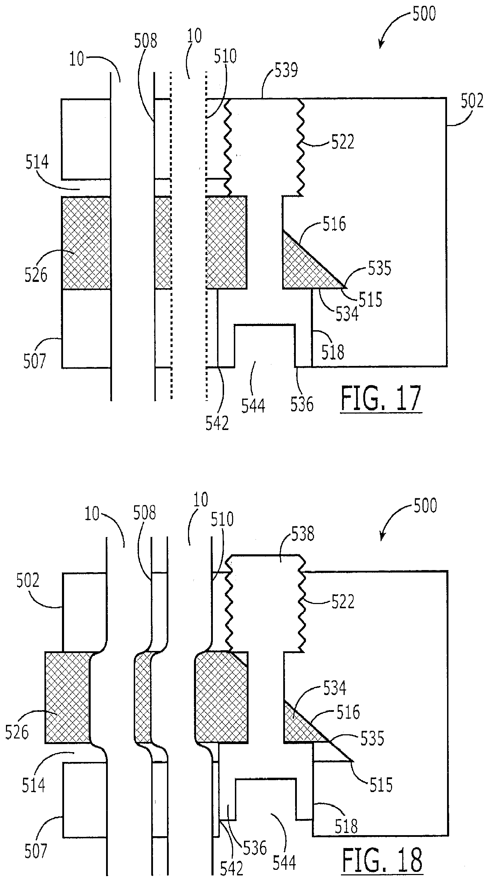

Figs. 17 - 18 sequentially depict the process by which the cable 10 is locked onto the crimp body 502. As noted earlier, a cable 10 is looped around a desired portion of bone to be stabilized and a device 500 which is, for example, factory assembled in the initial configuration shown in Fig. 17 is placed at a desired location adjacent to the bone. A proximal portion of the cable 10 is laid into the groove 508 and advanced distally therethrough until the enlarged diameter proximal end 12 engages the proximal face 504 of the crimp body 502. The distal portion of the cable 10 which has been looped around the target portion of bone is inserted into the cable lumen 510 via the proximal face 504 of the crimp body 502. The distal portion of the cable 10 is passed through the insert lumen 530 and out of the distal end of the cable lumen 510. The cable 10 is then connected to any known tensioning mechanism and tightened to the desired tension as would be understood by those skilled in the art. As shown in Fig. 18, the screw 536 is then tightened into the screw lumen 520 moving the shaft 540 of the screw 536 distally through the screw groove 532 until the distal end of the head 542 engages a proximal end of the insert 526. Further insertion of the screw 536 into the screw lumen 520 moves the insert 526 distally across the slot 514 causing the oblique end 534 to slide along the wall 516 moving the insert 526 laterally within the slot 514. This moves the insert groove 528 and the insert lumen 530 out of alignment with the groove 508 and the cable lumen 510, respectively, as shown in Fig. 18, deforming the portions of the cable received within the insert 526 and locking the cable 10 in position relative to the crimp body 502. As described above, this also locks the tension applied to the cable 10.

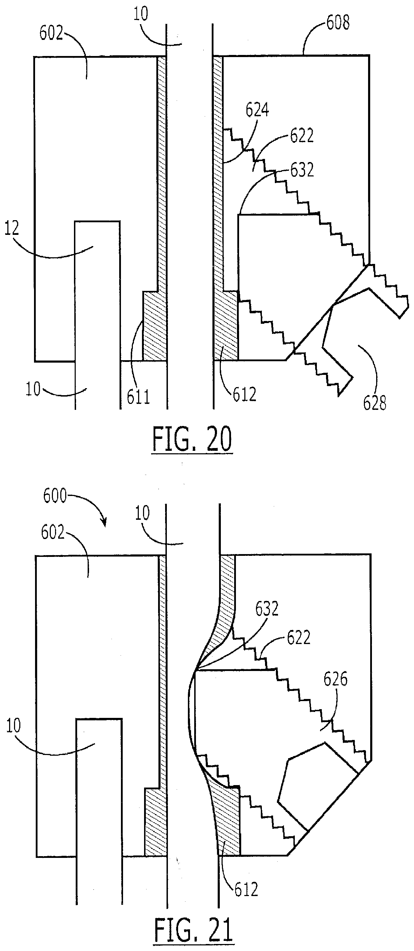

As shown in Figs. 20 and 21, a device 600 according to a further illustrative example includes a substantially cylindrical insert 612 secured to a crimp body 602 by a screw 626. The crimp body 602 includes a cable lumen 620 and a crimp lumen 610 extending longitudinally therethrough with the cable lumen 620 extending only partially through the length of the crimp body 602. In addition, the crimp body 602 includes an oblique wall 604 formed as a cut-out of one corner thereof with a screw hole 622 extending into the crimp body 602 via the wall 604 at an angle oblique relative to axes of the cable lumen 620 and the crimp lumen 610. In an exemplary embodiment, the screw hole 622is formed at an angle between approximately 0 - 89° with respect to the crimp lumen 610. A diameter of the crimp lumen 610 is selected to slidably receive the insert 612 therein (i.e., the inner diameter of the crimp lumen 610 is slightly larger than an outer diameter of the insert 612). Those skilled in the art will understand that, although the crimp lumen 610 and the insert 612 are shown as cylindrical in this embodiment, any shape may be employed so long as the insert is slidably insertable in the crimp lumen 610 and so long as the cable lumen 618 in the insert 612 is suitable to slidably receive the cerclage cable 10. A distal end of the insert 612 comprises an increased diameter head 614 which is housed in an increased diameter section 611 of the crimp lumen 610 with a shaft 616 extends therefrom to substantially traverse the crimp body 602. The screw hole 622 extends into the crimp body 602 and opens into the crimp lumen 610.

The cable lumen 620 extends into the crimp body 602 by a predetermined distance and permanently houses a first end 12 of the cable 10. Specifically, the first end 12 of the cable 10 may be factory installed into the cable lumen 620. Alternatively, the cable lumen 620 may extend longitudinally through the crimp body 602 and open to a proximal face 608 thereof. The first end 12 of the cable 10 may comprise an enlarged diameter and may lockingly engage the proximal face 608, as described with respect earlier embodiments. The screw 626 is insertable into the screw hole 622 and comprises a slot 628 at a distal end with a threaded shaft 630 extending proximally therefrom. The screw 628 may be formed in a manner substantially similar to the screw 320 of Figs. 7 - 9, with a proximal end of the screw 628 tapering to a tip 632, as described above.

Figs. 20 - 21 sequentially depict the process by which the cable 10 is locked around a target portion of bone using the crimp body 602. After the first end 12 of the cable 10 is secured within the recess 620, the free end of the cable 10 is looped around the target portion of bone until it is adjacent to the proximal surface 608 of the crimp body 602. The free end of the cable 10 is then inserted through the cable lumen 618 of the insert 612 which resides in crimp lumen 610 of the crimp body 602 and passed out of the distal face 606 of the crimp body 602. Tension is then applied to the free end of the cable 10 using any conventional mechanism until the desired tension is reached. While maintaining this tension, the screw 626 is tightened into the screw hole 622 until the proximal end thereof engages the outer surface of the insert 612. Further insertion of the screw 626 into the screw hole 622 deforms the insert 612 crushing it against the cable 10 and locking the cable 10 within the crimp body 602 at the selected tension, as shown in Fig. 21.

It will be apparent to those skilled in the art that various modifications and variations may be made in the structure and the methodology of the present invention, without departing from the scope of the invention. Thus, it is intended that the present invention cover modifications and variations of the invention provided that they come within the scope of the appended claims.

## Claims

1. An apparatus (100) for securing a cerclage member about a bone within a living body, the apparatus (100) comprising:
a crimp body (102) including a first lumen (108) extending longitudinally therethrough from a first proximal opening to a first distal opening, the first lumen (108) being sized and shaped to slidably receive a cerclage member (10) therein and a second lumen (110) extending substantially parallel to the first lumen (108) from a second proximal opening to a second distal opening; and
an insert (118) sized to be inserted into a distal portion of the second lumen (110), an insert lumen (132) extending through the insert (118) and aligning with the proximal portion of the second lumen (110) when the insert (118) is received within the distal portion of the second lumen 10), the insert (118) being crushable over the cerclage member (10) inserted therethrough to lock the cerclage member (10) relative to the crimp body (102) and maintaining a desired tension on the cerclage member (10), wherein a diameter of the second distal opening and of the distal portion of the second lumen (110) is greater than a diameter of a proximal portion of the second lumen (110);
**characterized in that**
a distal head (120) of the insert (118) includes an outer surface tapered from a maximum diameter at a proximal end thereof to a minimum diameter at a distal end thereof.

2. The apparatus of claim 1, further comprising a crushing member (134) sized for insertion into the distal portion of the second lumen (110), the crushing member (134) defining a tapered lumen (138) extending therethrough, the tapered lumen (138) being sized and shaped to receive the head (120) of the insert (118) therein so that advancing the crushing member (134) proximally over the head (120) of the insert (118) applies force radially inward crushing the head (120) of the insert (118) over a cerclage member (10) inserted therethrough.

3. The apparatus of claim 2, wherein an inner surface of a distal portion of the second lumen (110) is threaded and wherein an outer surface (136) of the crushing member (134) includes a corresponding thread oriented so that rotation of the crushing member (134) in a first direction advances the crushing member (134) proximally into the second lumen (110).

4. The apparatus of claim 3, wherein a distal end of the crushing member (134) includes a socket (142) configured to engage a tool (150) for rotatably driving the crushing member (134) proximally into the second lumen (110) over the head (120) of the insert (118).

5. The apparatus of claim 3, wherein the insert (118) includes a threaded proximal end sized to engage the thread of the distal portion of the second lumen (110).

6. The apparatus of any one of claims 1 to 5, the head (120) of the insert (118) being formed as a plurality of arms (128) separated from one another by a plurality of slots (130) extending proximally through the head (120).

7. The apparatus of any one of claims 1 to 6, wherein the first lumen (108) is formed as an open groove.

8. The apparatus of any one of claims 1 to 7, wherein the cerclage member (10) is a cable including an enlarged proximal end (12), the enlarged proximal end (12) being sized to prevent its passage into the first lumen (108).

## Patentansprüche

1. Vorrichtung (100) zum Befestigen eines Cerclage-Elements um einen Knochen in einem lebenden Körper, wobei die Vorrichtung (100) umfasst:
einen Crimpkörper (102) mit einem ersten Lumen (108), das sich in Längsrichtung von einer ersten proximalen Öffnung zu einer ersten distalen Öffnung hindurch erstreckt, wobei das erste Lumen (108) zum verschiebbaren Aufnehmen eines Cerclage-Elements (10) bemessen und geformt ist, und einem zweiten Lumen (110), das sich im Wesentlichen parallel zum ersten Lumen (108) von einer zweiten proximalen Öffnung zu einer zweiten distalen Öffnung erstreckt; und
einen Einsatz (118), der zum Einsetzen in einen distalen Abschnitt des zweiten Lumens (110) bemessen ist, ein Einsatzlumen (132), das sich durch den Einsatz (118) erstreckt und mit dem proximalen Abschnitt des zweiten Lumens (110) ausgerichtet ist, wenn der Einsatz (118) im distalen Abschnitt des zweiten Lumens (110) aufgenommen ist, wobei der Einsatz (118) über das darin eingeführte Cerclage-Element (10) eindrückbar ist, um das Cerclage-Element (10) relativ zum Crimpkörper (102) zu arretieren und eine gewünschte Spannung am Cerclage-Element (10) aufrechtzuerhalten,
wobei ein Durchmesser der zweiten distalen Öffnung und des distalen Abschnitts des zweiten Lumens (110) größer als ein Durchmesser eines proximalen Abschnitts des zweiten Lumens (110) ist:
**dadurch gekennzeichnet, dass**
ein distaler Kopf (120) des Einsatzes (118) eine Außenoberfläche aufweist, die sich von einem maximalen Durchmesser an dessen proximalem Ende zu einem minimalen Durchmesser an dessen distalem Ende verjüngt.

2. Vorrichtung nach Anspruch 1, ferner umfassend ein Quetschelement (134), das zum Einsetzen in den distalen Abschnitt des zweiten Lumens (110) bemessen ist, wobei das Quetschelement (134) ein sich dadurch erstreckendes, verjüngendes Lumen (138) definiert, wobei das sich verjüngende Lumen (138) zum Aufnehmen des Kopfs (120) des Einsatzes (118) darin so bemessen ist, dass ein Vorschub des Quetschelements (134) proximal über den Kopf (120) des Einsatzes (118) eine Kraft radial nach innen ausübt, die den Kopf (120) des Einsatzes (118) über ein dadurch eingesetztes Cerclage-Element (10) eindrückt.

3. Vorrichtung nach Anspruch 2, wobei eine Innenfläche eines distalen Abschnitts des zweiten Lumens (110) ein Gewinde aufweist, und wobei eine Außenfläche (136) des Quetschelements (134) ein entsprechendes Gewinde aufweist, das so ausgerichtet ist, dass eine Drehung des Quetschelements (134) in eine erste Richtung das Quetschelement (134) proximal in das zweite Lumen (110) schiebt.

4. Vorrichtung nach Anspruch 3, wobei ein distales Ende des Quetschelements (134) eine Hülse (142) umfasst, die zum Eingriff eines Werkzeugs (150) zum drehbaren Antreiben des Quetschelements (134) proximal in das zweite Lumen (110) über den Kopf (120) des Einsatzes (118) konfiguriert ist.

5. Vorrichtung nach Anspruch 3, wobei der Einsatz (118) ein proximales Gewindeende aufweist, das so bemessen ist, dass es mit dem Gewinde des distalen Abschnitts des zweiten Lumens (110) in Eingriff steht.

6. Vorrichtung nach einem der Ansprüche 1 bis 5, wobei der Kopf (120) des Einsatzes (118) als Vielzahl von Armen (128) ausgebildet ist, die durch eine Vielzahl von Schlitzen (130) voneinander getrennt sind, die sich proximal durch den Kopf (120) erstrecken.

7. Vorrichtung nach einem der Ansprüche 1 bis 6, wobei das erste Lumen (108) als offene Nut ausgebildet ist.

8. Vorrichtung nach einem der Ansprüche 1 bis 7, wobei das Cerclage-Element (10) ein Kabel mit einem vergrößerten proximalen Ende (12) ist, wobei das vergrößerte proximale Ende (12) so bemessen ist, dass sein Durchtritt in das erste Lumen (108) verhindert wird.

## Revendications

1. Appareil (100) destiné à arrimer un élément de cerclage autour d'un os dans un corps vivant, l'appareil (100) comprenant :
un corps à sertir (102) incluant une première lumière (108) s'étendant longitudinalement à travers celui-ci d'une première ouverture proximale à une première ouverture distale, la première lumière (108) étant dimensionnée et façonnée de manière à recevoir à l'intérieur par coulissement un élément de cerclage (10), et une seconde lumière (110) s'étendant en grande partie parallèlement à la première lumière (108) d'une seconde ouverture proximale à une seconde ouverture distale, et
un insert (118) dimensionné de manière à être introduit dans une portion distale de la seconde lumière (110), une lumière d'insert (132) s'étendant à travers l'insert (118) et s'alignant avec la portion proximale de la seconde lumière (110) lorsque l'insert (118) est reçu dans la portion distale de la seconde lumière (110), l'insert (118) pouvant être écrasé par-dessus l'élément de cerclage (10) introduit à travers lui afin de bloquer l'élément de cerclage (10) par rapport au corps à sertir (102) et maintenant une tension souhaitée sur l'élément de cerclage (10),
dans lequel un diamètre de la seconde ouverture distale et de la portion distale de la seconde lumière (110) est supérieur à un diamètre d'une portion proximale de la seconde lumière (110),
**caractérisé en ce qu'**une tête distale (120) de l'insert (118) inclut une surface extérieure présentant un amincissement progressif allant d'un diamètre maximal sur une extrémité proximale de celle-ci à un diamètre minimal sur une extrémité distale de celle-ci.

2. Appareil selon la revendication 1, comprenant en outre un élément d'écrasement (134) dimensionné pour une introduction dans la portion distale de la seconde lumière (110), l'élément d'écrasement (134) définissant une lumière à amincissement progressif (138) s'étendant à travers lui, la lumière à amincissement progressif (138) étant dimensionnée et façonnée pour recevoir à l'intérieur la tête (120) de l'insert (118) de sorte qu'une progression de l'élément d'écrasement (134) de manière proximale par-dessus la tête (120) de l'insert (118) applique une force radialement vers l'intérieur écrasant la tête (120) de l'insert (118) par-dessus un élément de cerclage (10) introduit à travers lui.

3. Appareil selon la revendication 2, dans lequel une surface intérieure d'une portion distale de la seconde lumière (110) est filetée, et dans lequel une surface extérieure (136) de l'élément d'écrasement (134) inclut un filet correspondant orienté de sorte que la rotation de l'élément d'écrasement (134) dans une première direction fasse avancer l'élément d'écrasement (134) de manière proximale jusque dans la seconde lumière (110).

4. Appareil selon la revendication 3, dans lequel une extrémité distale de l'élément d'écrasement (134) inclut une emboîture (142) configurée pour venir en prise avec un outil (150) pour entraîner d'une manière permettant la rotation l'élément d'écrasement (134) de manière proximale jusque dans la seconde lumière (110) par-dessus la tête (120) de l'insert (118).

5. Appareil selon la revendication 3, dans lequel l'insert (118) inclut une extrémité proximale filetée dimensionnée pour venir en prise avec le filet de la portion distale de la seconde lumière (110).

6. Appareil selon l'une quelconque des revendications 1 à 5, la tête (120) de l'insert (118) se présentant sous la forme d'une pluralité de bras (128) séparés les uns des autres par une pluralité de fentes (130) s'étendant de manière proximale à travers la tête (120).

7. Appareil selon l'une quelconque des revendications 1 à 6, dans lequel la première lumière (108) est façonnée sous la forme d'une rainure ouverte.

8. Appareil selon l'une quelconque des revendications 1 à 7, dans lequel l'élément de cerclage (10) est un câble incluant une extrémité proximale agrandie (12), l'extrémité proximale agrandie (12) étant dimensionnée pour empêcher son passage jusque dans la première lumière (108).
